# EUROPEAN PATENT APPLICATION

(11) **EP 2 394 520 A1**
(43) Date of publication of application: **14.12.2011**
(21) Application number: 10738666.6
(22) Date of filing: 03.02.2010
(51) Int. Cl.: A24F 47/00, A61M 15/06

(54) **NON-COMBUSTION SMOKING JIG**

(30) Priority: 07.02.2009 JP 2009049879
(71) Applicant: Shimizu, Kazuhiko, Kobe-shi, Hyogo 651-1303 (JP)
(72) Inventor: Shimizu, Kazuhiko, Kobe-shi, Hyogo 651-1303 (JP)
(74) Representative: Kindermann, Peter
(86) International application number: PCT/JP2010/052002
(87) International publication number: WO 2010/090338

(57) **Abstract**

In order to use a commercially available cigarette or cigar as it is, vaporize nicotine from the tobacco leaves, and enable inhalation of the vaporized nicotine without burning the product, a non-combustion smoking tool comprises a slender heater 1 having a sharp end which is directly inserted into a commercially available cigarette or cigar 100 to directly heat the leaves of the cigarette or cigar by the heater so as to vaporize nicotine contained in the tobacco leaves and enable inhalation of the vaporized nicotine.

## Description

### TECHNICAL FIELD

The present invention relates to a tool for allowing smoker to inhale nicotine vaporized from tobacco leaves by directly heating the tobacco leaves without combusting the tobacco leaves.

### BACKGROUND ART

Tobaccos include chewing tobacco, snuffing tobacco, cigarette, cigar and so on, in which the cigarette and the cigar allow vaporization and inhalation of nicotine, tar and other components contained in tobacco leaves by lighting the tobacco leaves and combusting the tobacco leaves. The temperature of the burning portion reaches 600 to 900°C. Many fires are reported to be caused by smoking in bed, throwing burning cigarette, and so on.

In recent years, many cigarettes are smoked because of the easiness of handling. However, when a cigarette is lighted for smoking, it is known that the tobacco leaves are burned, and at the same time the wrapping paper containing many chemicals is burned, so that the tobacco smoke contains many harmful materials to human bodies, other than nicotine, as represented by tar, carbon monoxide, acetaldehyde, acrolein, benzopyrene, and so on. In recent years, many filter cigarettes are smoked in order to reduce adverse effects on human bodies due to the smoking. Various ideas have been incorporated into the filter to absorb the harmful materials to human bodies as represented by nicotine, tar, carbon monoxide, acetaldehyde, acrolein, benzopyrene, and so on so as to prevent much of these harmful materials from entering human bodies of smokers who smoke the filter cigarettes.

However, although the harm of tobacco to a smoker smoking a filter cigarette and thus inhaling smoke with a reduced level of these harmful materials is reduced, bad effects of so-called passive smoking have been pointed out that people around the smoker are forced to directly smoke the smoke (secondary smoke) rising from the burning portion, and thus to directly inhale the smoke containing these harmful materials. As a result, in recent years, smoking is prohibited in companies, in tea rooms and restaurants, in public transportation including airplanes, trains, buses and taxis, in public places including roads, airports, stations, and so on, so that there are fewer and fewer places where smokers can smoke. Furthermore, smoking causes that a room or car where the smoking has taken place is filled with specific smell of tobacco smoking, and very unpleasant smell for non-smokers remains in the room or car.

In addition, when one wants to smoke during work, it is required to go to a specific smoking place for smoking. It is said that work stops during such time, essentially reducing work time, thus reducing work efficiency It is said that the reason why a smoker cannot quit smoking regardless of such bad effects is because the body of smoker is already nicotine-addicted, so that the amount of nicotine in the body decreases by not smoking for a certain period of time, causing withdrawal symptoms, and that the desire of the body to ease the withdrawal symptoms makes quitting smoking difficult.

Thus, non-combustion smoking devices to heat only tobacco leaves for inhalation of nicotine and so on contained in the tobacco leaves have been devised since pretty long time ago.

Patent Document 1 discloses a non-combustion device as set forth below. The invented product has a small container formed in a mouthpiece portion, and is designed so that tobacco leaves can be filled in the container, and the container can be electrically heated to a temperature to vaporize necessary components. This is designed to allow vaporization of nicotine and so on from the tobacco leaves when heated hot air passes through in the tobacco leaves filled in the container. The components vaporized from the tobacco leaves and the "hot air" are introduced through the mouthpiece into the respiratory organ of a person using the device.

Patent Document 2 discloses a non-combustion smoking tool which allows tobacco leaves to be filled in a container and electrically produces "hot air", and which introduces the "hot air" into the container and allows it to pass through in the tobacco leaves filled in the container when a smoker inhales, whereby the "hot air" heats the tobacco leaves to vaporize nicotine and so on from the tobacco leaves, making it possible to inhale nicotine along with the "hot air".

However, these non-combustion smoking tools cause inhalation of nicotine and so on along with "hot air", thus causing the mouth of the inhaler to be hot inside. In order to improve this problem, these tools are devised to take in outside air, and thus devised to prevent the smoker from feeling hot in the mouth by taking in "cold outside air" and "hot air containing nicotine and so on" at the same time.

Further, in Patent Document 3 and Patent Document 4, there are concepts of tools in which a rod shaped case having nicotine and flavor material impregnated therein instead of real tobacco leaves is prepared, and the case is heated to vaporize the nicotine and flavor material filled in the case, making it possible to take in these vapors. However, these vapors are not nicotine and so on from real tobacco leaves, so that they are very far from the flavor of real tobacco leaves.

Furthermore, in Patent Document 5, there is a concept of smoking tool in which a rod shaped chamber is prepared, and two kinds of metals are put in the rod shaped chamber, while tobacco leaves are wrapped around the rod shaped chamber for use. This tool is heated by an electric current flowing in the two metals. When the rod shaped chamber is heated, the tobacco leaves wrapped around the chamber are heated to allow nicotine and so on vaporized from the tobacco leaves to be inhaled. However, inhalation of a general commercial cigarette or cigar, as it is, is not possible with this tool.

### PRIOR ART DOCUMENTS

### Patent Documents

Patent Document 1: US Patent 2,104,266
Patent Document 2: US Patent 4,141,369
Patent Document 3: Japanese Translation of PCT Application 2006-525798
Patent Document 4: Japanese Patent 3,012,253
Patent Document 5: US Patent 5,285,798

### SUMMARY OF THE INVENTION

### PROBLEM TO BE SOLVED BY THE INVENTION

The present invention is proposed in view of such conventional circumstances, and the object is to devise a tool which, while using a general commercial cigarette or cigar as it is, can vaporize nicotine from tobacco leaves at a suitable temperature to enable smoker to inhale the nicotine without combusting the tobacco leaves, making it possible to inhale it.

### MEANS FOR SOLVING THE PROBLEM

In order to solve the above-described problems, a slender heater having a sharp end is inserted into a commercial cigarette or cigar to directly heat only tobacco leaves so as to vaporize nicotine from the tobacco leaves at a relatively low temperature and enable its inhalation.

### EFFECT OF THE INVENTION

While products of prior inventions cannot be used without filling tobacco leaves in a container or forming the tobacco leaves in a sheet shape, the present invention has an advantage to allow use of a commercial cigarette or cigar as it is, which is practically very convenient.

Further, the prior inventions heat tobacco leaves by allowing "hot air" to pass through in the container filled with the tobacco leaves. However, the present invention has an advantage that by inserting a heater into a commercial cigarette or cigar and supplying heat from the heater directly to the tobacco leaves, it is possible to vaporize nicotine from the tobacco leaves at a relatively low temperature (70°C to 230°C) and supply nicotine from the real tobacco leaves to the smoker without producing harmful tar, carbon monoxide, benzopyrene, and so on that are produced at the time of lighting tobacco for smoking and are inhaled into the body.

Further, the present invention also has an advantage that since the heater inserted in the real cigarette or cigar directly heats the tobacco leaves, the heat utilization efficiency increases, making it possible to use a relatively small heater, and reducing the amount of electric power used, so that if a battery is used as a power supply, it can be a small battery, resulting in that the shape of the tool can be made small so as to increase the portability of the tool.

Further, the present invention also has an advantage that since the heating portion is located near the center of the cigarette, the temperature of heating the wrapping paper containing many chemicals is lowered, thereby enabling reduction of the vaporization of harmful chemicals from the wrapping paper.

Further, the present invention also has an advantage that since the heating portion is located near the center of the cigarette, the heat transfer to the outer case is reduced, resulting in that the outside temperature of the tool is low, thereby preventing the smoker even holding the tool of the present invention by hand from feeling hot.

Further, the present invention also has an advantage that even when a commercial cigarette or cigar is inserted into the tool of the present invention, the shape of the cigarette or cigar does not change, so that it is possible to take a cigarette or cigar out of the tool midway during smoking of the cigarette or cigar using the tool, move to a smoking-permitted place, and light the cigarette or cigar taken out, so as to enable normal smoking by lighting.

Further, according to the present invention, a commercial cigarette or cigar is used in the form as it is, so that a smoker can dispose of the cigarette or cigar every time it smokes. Thus, there is an advantage that even if the smoking tool was used by others, and if a person having used the tool in advance has germs, there is no concern of oral infection because what the mouth of the smoker touches are only the cigarettes or cigars which the smoker has.

Further, the present invention also has an advantage that there is nothing to be disposed of after inhalation of nicotine, other than commercial cigarettes or cigars from which nicotine has been removed and which are to be disposed of. Thus, these materials can be easily burned up, which is environment-friendly as well.

Further, the present invention also has an advantage that because of non-use of fire, there is no danger of causing fire due to smoking, so that it is possible to take in nicotine from tobacco leaves even in places where open flame is prohibited, such as where gasoline is handled.

Further, a combination of the product of the present invention and a portable game machine makes it possible to inhale nicotine of tobacco leaves in public transportation such as airplanes, trains, buses, taxis and so on where no smoking is required, so that even heavy smokers do not feel pain during long hour journeys and can enjoy comfortable journeys.

Further, at work in general companies, if the product of the present invention is combined with, for example, a machine tool, a soldering iron or an office equipment such as a telephone set, or is placed on a table in e.g. an office room or a conference room, it is possible to inhale nicotine from tobacco leaves even during work, during business or during conference, so that there is no need to leave the place of duty for "smoking" during work, during business or during conference, thus significantly increasing work efficiency.

Further, in general homes, smokers cannot smoke in the room, which leads to smoking on a cold balcony (who are called "firefly tribe" in Japan). However, the use of the product of the present invention makes it possible to smoke in the room because there is no smell attached to the room.

### BRIEF DESCRIPTION OF THE DRAWINGS

FIG. 1 is an outline view of a non-combustion smoking tool of the present embodiment (Example 1).
FIG. 2 is an A-A' cross-sectional view of the non-combustion smoking tool of the present embodiment.
FIG. 3 is a B-B' cross-sectional view of the non-combustion smoking tool of the present embodiment.
FIG. 4 is a cross-sectional view when a filter cigarette is inserted according to the present embodiment.
FIG. 5 is a cross-sectional view of a heater used in the tool of the present application (Example 2).
FIG. 6 is an enlarged view of an end of the heater used in the tool of the present application.
FIG 7 is a cross-sectional view of a stopper used in the tool of the present application (Example 5).
FIG 8-1 is an outline view of another embodiment of a tool of the present application (Example 6).
FIG 8-2 is a D-D' cross-sectional view of FIG. 8-1.
FIG. 8-3 is an E-E' cross-sectional view of FIG. 8-1.
FIG 9 is an outline view of another embodiment of a tool of the present application (Example 7).

### MODES FOR CARRAYING OUT THE INVENTION

Hereinafter, detailed description will be made with reference to the drawings, giving a filter cigarette as an example of commercial cigarettes and cigars for the present invention.

### EXAMPLE 1

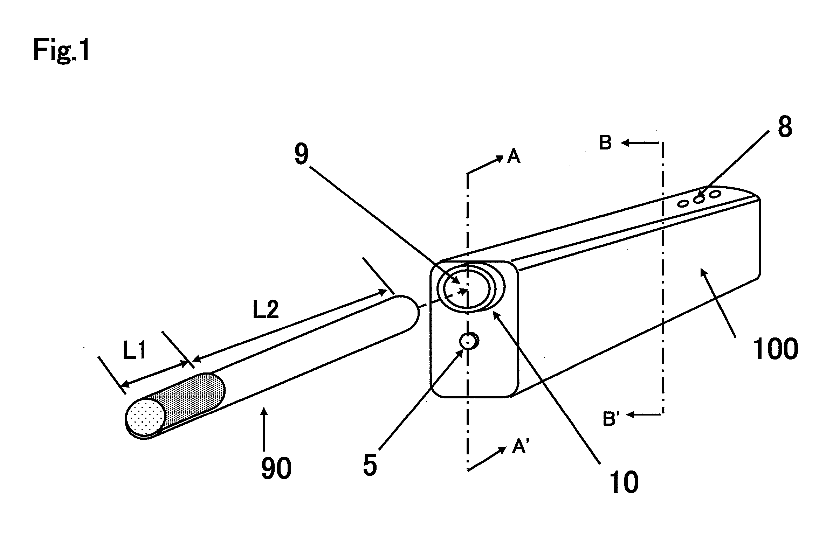

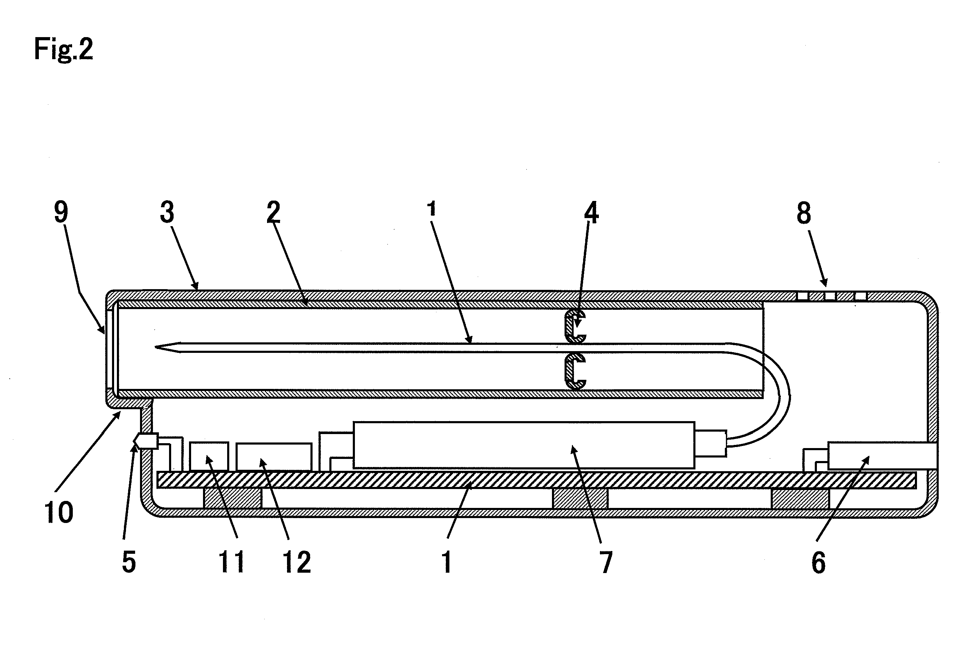

FIG 1 shows appearance of an example of a tool according to the present invention. A tool 100 comprises: a tobacco insertion opening 9 for inserting a filter cigarette 90; a projection 10; an indicator 5 for indicating an operation state; a connector 6 (shown in FIG. 2) for receiving electric power from outside; an outside air inlet 8; and inner constituents shown in FIG. 2.
FIG. 2 is a view for explaining the inner structure along section line A-A' of FIG 1. The tool of the present invention basically comprises: a slender heater 1 with a heater sleeve 7; a thermal insulation pipe 2 for preventing heat of the heater 1 from being transferred to an outer case 3; the outer case 3 with the outside air inlet 8; a stopper 4 for fixing an insertion stop position of the filter cigarette; the indicator 5 for indicating the operation state of the tool; and the connector 6 for receiving electric power from outside. The heater sleeve 7 includes a sealing portion 28 for securing long-term reliability of the heater 1. A printed circuit board 13 has mounted thereon at least the connector 6, the indicator 5, the heater 1, a control circuit A11 of the indicator 5, and a control circuit B of the heater 1. Here, an end of the heater 1 is designed to be positioned slightly inside the tobacco insertion opening 9, which is considered in order to prevent a smoker using the tool of the present application from getting burned.

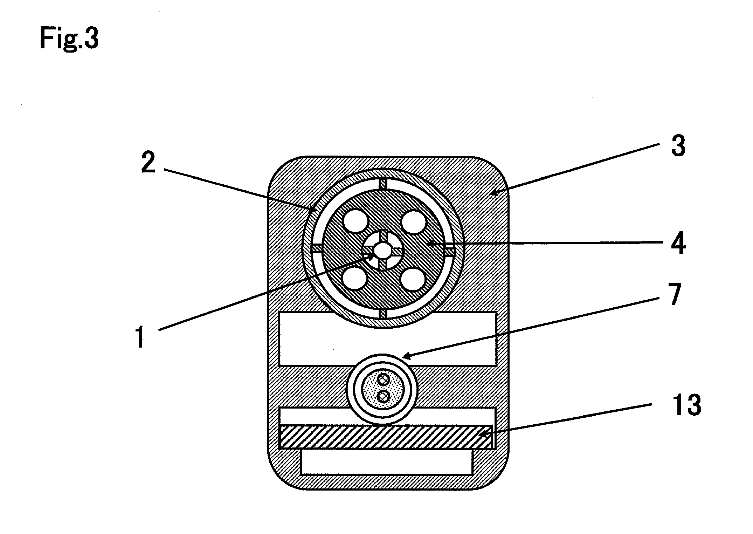

FIG 3 is a view for explaining the inner structure along section line B-B' of FIG 1. The outer case 3 is formed and shaped to hold the thermal insulation pipe 2, the heater sleeve 7 and the printed circuit board 13, which are placed internally.

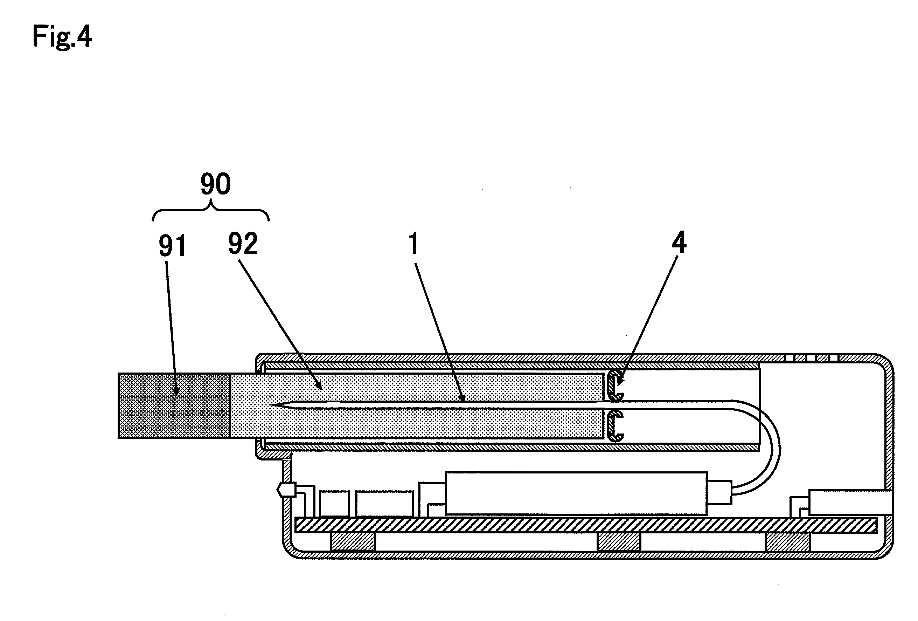

Regarding how to use the tool 100 according to the present invention, it is used in a procedure described below.
1. A smoker inserts its favorite filter cigarette 90 into the cigarette insertion opening 9 of the tool 100 up to where it is stopped by the stopper 4. This allows the slender heater 1 to be inserted into a tobacco portion 92 of the filter cigarette 90. FIG. 4 shows a positional relationship between the tool of the present application in this state of insertion and the inserted filter cigarette 90.
2. The smoker waits until electric power is supplied through the connector 6 of the tool of the present application to raise the temperature of the heater 1 so as to directly heat the leaves of the tobacco portion 92 and vaporize nicotine. Here, the indicator 5 may inform that it has become possible to inhale, or may simply indicate only the heating state.
3. When the tobacco leaves are heated, and vaporization of nicotine from the tobacco leaves starts, the smoker inhales nicotine vaporized from the tobacco leaves through a filter 91, either while the filter cigarette 90 is kept inserted in the tool of the present application, or after the filter cigarette 90 is taken out of the tool of the present application.
4. Thereafter, steps 2 and 3 are repeated until the vaporization of nicotine from the tobacco leaves of the filter cigarette 90 stops.

Another procedure to use the tool 100 of the present application is described below.
1. A smoker sets the stopper 4 at a front position in advance. For example, it is set at a position to allow insertion of about 1/3 of a tobacco portion 92. The smoker supplies electric power to the tool of the present application through the connector 6 to heat the heater.
2. The smoker inserts its favorite filter cigarette 90 into the cigarette insertion opening 9 of the tool 100 up to where it is stopped by the stopper 4. This allows the slender heater 1 to be inserted into about 1/3 of the tobacco portion 92.
3. When the tobacco leaves are heated, and vaporization of nicotine from the tobacco leaves starts, the smoker inhales nicotine vaporized from the tobacco leaves through a filter 91.
4. When the smoker inhales and feels that the vaporization of nicotine from the tobacco leaves has stopped, the smoker moves the stopper to a further rear 1/3 position and sets it at such position either by using the filter cigarette or by using a stopper position change button 44. This allows tobacco leaves of a new portion of the inserted filter cigarette 90 to be heated, resulting in that nicotine is newly vaporized from the tobacco leaves of such portion.
5. Further, when the smoker inhales and feels that the vaporization of nicotine from the tobacco leaves has stopped, the smoker sets the stopper at a further rear 1/3 position so as to allow tobacco leaves of a new portion to be heated, so that nicotine is newly vaporized from the tobacco leaves of such portion.
6. This way of inhalation allows the smoker to inhale nicotine for a long time even with a single filter cigarette 90.

### EXAMPLE 2

Hereinafter, a slender heater 1 having a sharp end to be used for the present invention will be described in detail.

Approximate dimensions of a general filter cigarette 90 are tobacco portion L2=58mm, filter portion L1=26mm, and diameter=8mmΦ.

Insertion of a slender heater into the filter cigarette 90 is not easy. First of all, it is required to have a sharp end as a condition.

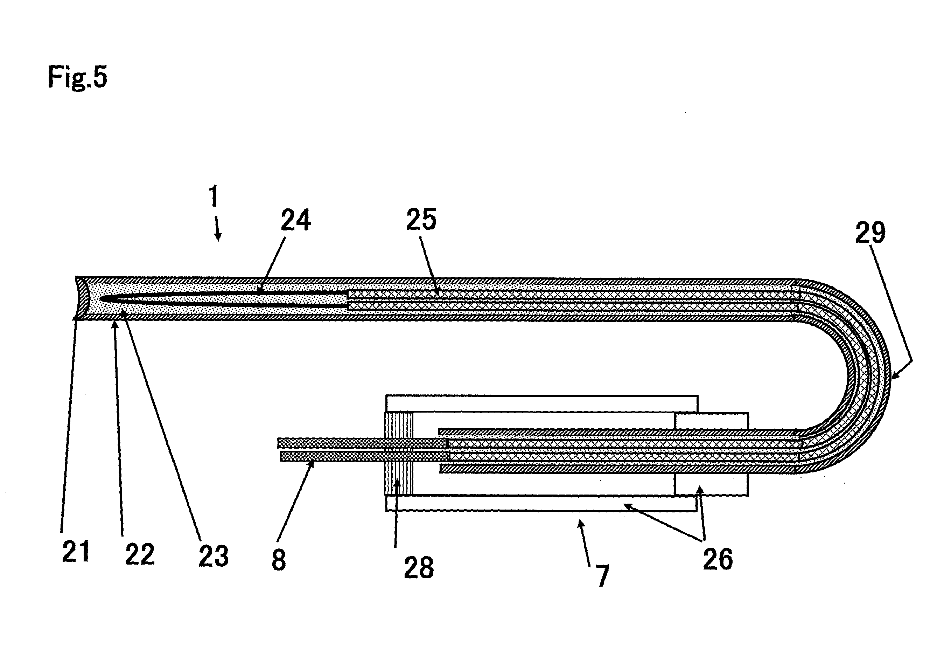

FIG. 5 shows a cross-sectional view of an inner structure of a general slender heater 1. A metal tube 22 is sealed with an end sealing portion 21 to prevent moisture from entering inside. The metal tube 22 has inside a resistance heating wire 24 (which portion is heated to serve as a heater) and an inner lead wire 25 connected to the resistance heating wire 24 by welding, and contains magnesium oxide powder 23 filled inside. The inner lead wire 25 which extends from the heater sleeve 7 is in turn connected on the outside to a lead wire 8 made of a solderable wire. While the end sealing portion 21 is welded to the metal tube 22 by metal, the sealing portion 28 is sealed by glass, so that when the heater 1 is used, it is required to be used at a temperature of the sealing portion 28 not higher than 150°C. If it is used at 150°C or higher, it causes cracks in the glass sealing portion 28, and moisture enters inside to react with MgO, reducing insulation of MgO.

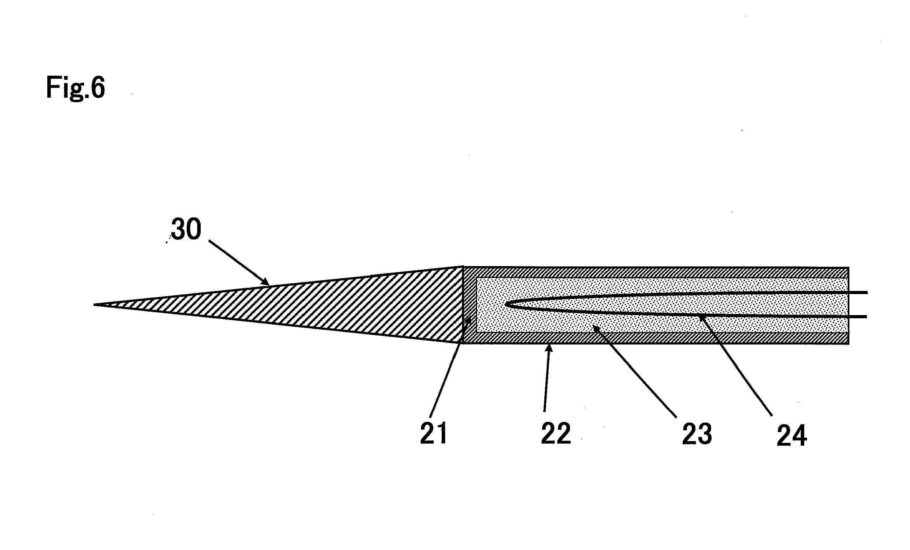

The sealing portion 21 of the heater 1 is substantially flat in structure, so that it cannot be easily inserted into the filter cigarette 90 as it is. Thus, the present inventor welded an end 30 made of metal to the end sealing portion 21 as shown in FIG. 6 so as to sharpen the end of the heater. Although this makes it possible to easily insert the heater 1 into the filter cigarette 90, a problem still remains that if the diameter of the heater 1 increases, it cannot be inserted.

Thus, the present inventor made experiments to find heater diameters to enable easy insertion into the filter cigarette 90.

The results as shown in Table 1 are the experimental results obtained.

**[Table 1]**

| | | |
|---|---|---|
| 1 | Φ=1.0 | Smooth insertion is possible. |
| 2 | Φ=1.5 | Smooth insertion is possible. |
| 3 | Φ=1.7 | Smooth insertion is possible. |
| 4 | Φ=1.9 | Smooth insertion is possible. |
| 5 | Φ=2.1 | Insertion is possible although with a little resistance. |
| 6 | Φ=2.3 | Insertion is possible although with resistance. |
| 7 | Φ=2.5 | Insertion resistance is high. Deformed outline is observed. |
| 8 | Φ=2.7 | Insertion is difficult. Forced insertion sometimes tears the paper. |

From the above experimental results, it has been found that the diameter of the heater 1 is required to be Φ=2.3mm or smaller to enable a smoker to smoothly insert a filter cigarette 90 into the tool of the present application.

### EXAMPLE 3

Next, tobacco leaves of a real filter cigarette were taken out, and the components vaporized from the tobacco leaves were analyzed, using a gas chromatography mass spectrometer, by putting only the tobacco leaves in a vessel and heating the vessel. Here, a focus was placed on the amount of nicotine produced among the components vaporized from the tobacco leaves, and its data were obtained. Table 2 shows the results. The heating time was 5 minutes.

**[Table 2]**

| | | | |
|---|---|---|---|
| 1 | Heating temperature=70°C | 1000µg | |
| 2 | Heating temperature=150°C | 3600µg | |
| 3 | Heating temperature=200°C | 4300µg | |
| 4 | Heating temperature=230°C | 4300µg | |
| 5 | Heating temperature=250°C | 3060µg | Leaves get combusted. |
| 6 | Heating temperature=280°C | 1027µg | Leaves get combusted |

An encyclopedia says that nicotine is oily liquid with a boiling point (decomposition) of 247°C. The reduction in the analyzed data amount when the heating temperature reaches 250°C is presumably because decomposition of nicotine vaporized out of the tobacco leaves by heating has started.

By further observing the appearance of the tobacco leaves, it has been found that the temperature is required to be 230°C or lower.

### EXAMPLE 4

On the other hand, since a smoker may use the tool of the present application by holding it by hand, it is required to be devised to prevent the outer case 3 from getting hot with the heat of the heater 1 transferred to the outer case 3.

Further, in order to make the finished outer case 3 compact, the heater 1 is required to be bent into U-shape for use as shown in FIG. 5. However, this structure causes a new problem that the heater 1 gets close to the heater sleeve 7 so as to raise the temperature of the sealing portion 28 in the sleeve 7 by the heat of the heater 1, making it impossible to secure long-term reliability of the heater 1.

In order to solve this problem, the present inventor has provided a thermal insulation pipe 2 (an example being a glass pipe or a ceramic pipe) for the heater 1. The use of the thermal insulation pipe 2 has made it possible to obtain the following advantages:
- It is difficult for the heat of the heater 1 to be transferred to the outer case 3, enabling the smoker to hold the tool of the present application for inhalation.
- It is difficult for the heat of the heater to be transferred to the sealing portion 28, making it possible to secure reliability of the heater.
- When inserting a filter cigarette 90 into the tool of the present application, the thermal insulation pipe 2 serves as a guide for insertion.
- Further, when the filter cigarette 90 is inserted into the tool of the present application, the heater 1 pushes out the inserted tobacco leaves, allowing the filter cigarette 90 to be larger in outer diameter and difficult to be drawn out of the tool of the present application.

In order to achieve these advantages, it has been found as a result of experiments that the optimum inner diameter of the thermal insulation pipe 2 is the outer diameter of the filter cigarette 90 plus 0.01 to 2.0mm.

### EXAMPLE 5

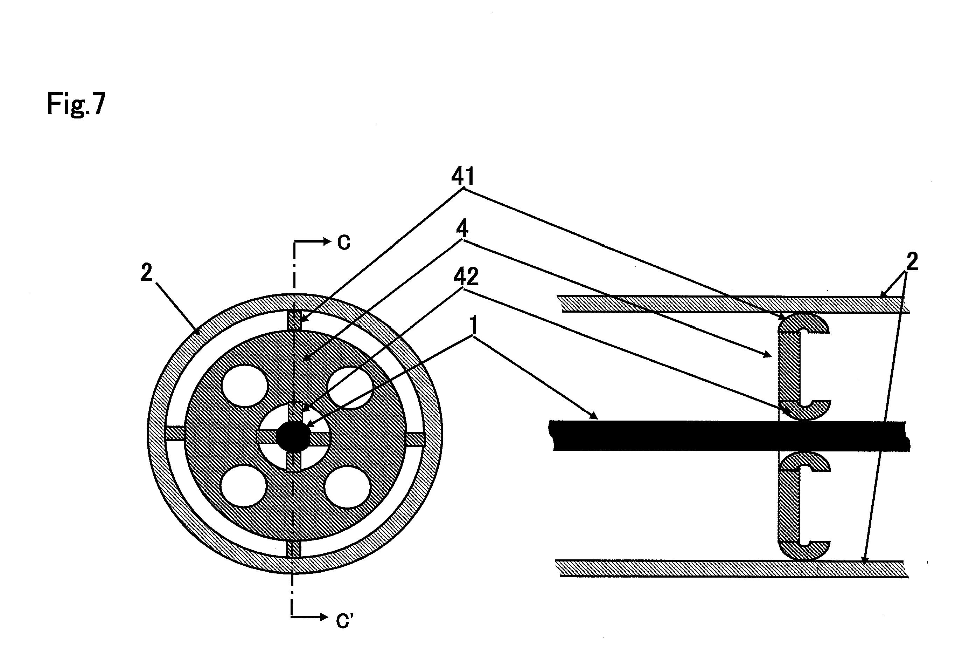

Next, a stopper 4 to be used for the tool of the present application will be described. FIG. 7 shows an embodiment example of the stopper 4. The stopper 4 is disc shaped and has outer claws 41 having spring properties as well as inner claws 42 having spring properties, to respectively generate frictional forces between the springs and the thermal insulation pipe 2 as well as the heater 1, so as to be fixed at an arbitrary position. According to the tool of the present application, the stopper 4 can be stopped at a predetermined position as a fixing position e.g. by inserting a slender rod through the tobacco insertion opening 9.

### EXAMPLE 6

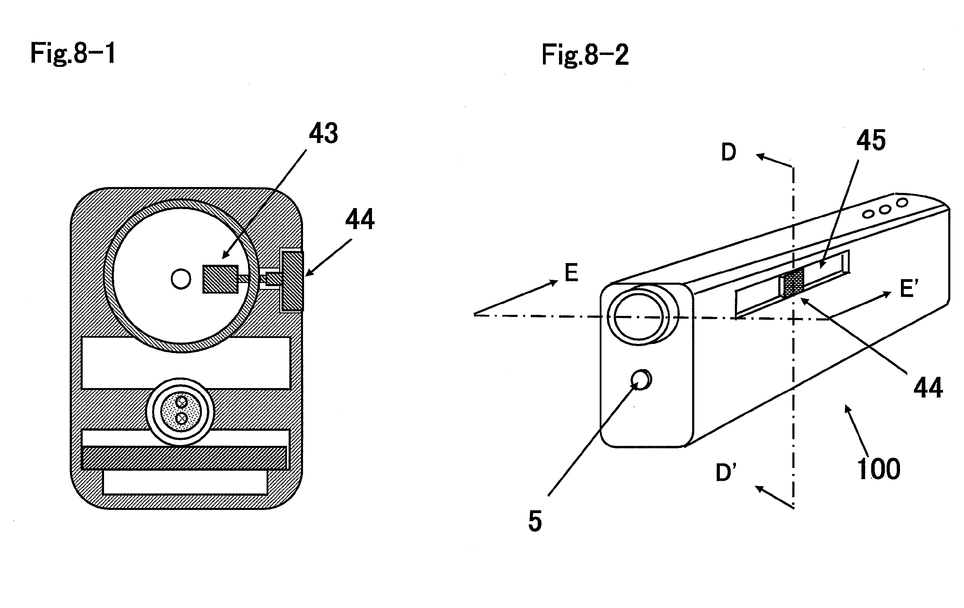

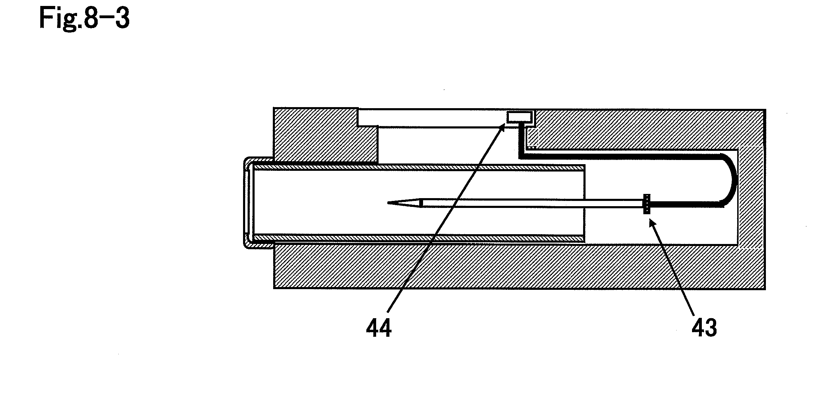

FIGs. 8 show a structure of a stopper 4 according to another example. In order to use the stopper of the present example, a groove 45 is formed in a side of an outer case 3 of a tool 100, while a space is formed between the outer case 3 and a thermal insulation pipe 2. A stopper 43 shown in FIG. 8-2 and FIG. 8-3 is inserted into this space. It is not necessary to explain that the positioning of the stopper is performed by a stopper positioning knob 44.

### EXAMPLE 7

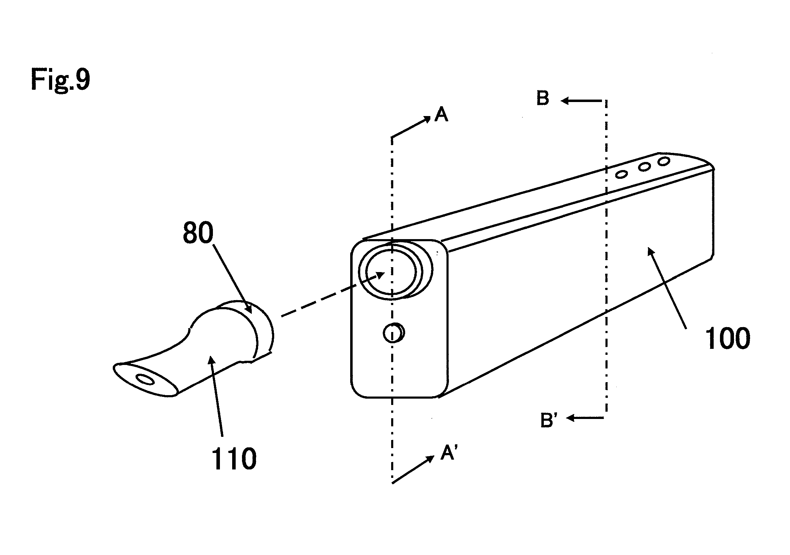

FIG. 9 shows Example 7. Nicotine was inhaled using a tool of the present application, and actually using a filterless cigarette and a cigar. In order to prevent a smoker using the tool of the present application from getting burned, the end 30 of the heater 1 does not project outside the tobacco insertion opening 9, causing a problem that all the tobacco leaves cannot be heated when the tool of the present application is used for a filterless cigarette or a cigar. More specifically, the problem is that about half of the tobacco leaves cannot be heated when the filterless cigarette or the cigar is used in the tool of the present application, because the tobacco leaves outside the tobacco insertion opening 9 cannot be heated.

Thus, the present inventor has devised a mouthpiece 110 which can be attached to the tool of the present application. The present mouthpiece 110, while having a shape similar to a mouthpiece portion of a normal smoking pipe, has an end portion 80 having an inner structure to fit to the projection 10 of the tool 100 of the present application. This structure can be any structure invented so far for a fitting method. The attachment of such mouthpiece 110 to the tool of the present application makes it possible to insert the entire length of the filterless cigarette or the cigar into the tool of the present application, making it possible to heat all the tobacco leaves. Further, the intake rate of the amount of nicotine vaporized from the tobacco leaves can be changed by inserting a filter into the mouthpiece 110 as is evident from the function of the filter.

In the Examples of the present application, examples have been described in which the electric power for heating the heater 1 is supplied through the connector 6 from outside. However, it is evident that a disposable battery, if used as an electric power, can be built in the tool of the present application. It is also possible to build in a rechargeable battery to recharge using the connector 6.

Further, the structure of the tool of the present invention is not limited to those described above. It is evident that the present invention covers a heater 1 which is not bent midway and has a straight shape for use, which only causes the tool 100 to have an elongated shape.

In addition, various structural items to allow a slender heater 1 to be directly inserted into a commercial cigarette or cigar can be devised from the tool of the present invention, not limited to the structures described above.

### INDUSTRIAL APPLICABILITY

The present invention makes it possible to use a commercial filter cigarette or cigar in the form as it is, and to inhale nicotine contained in the tobacco leaves without burning it.

### DESCRIPTION OF THE REFERENCE NUMERALS

- 1: Heater
- 2: Thermal insulation pipe
- 3: Outer case
- 4: Stopper
- 5: Indicator
- 6: Connector
- 7: Heater sleeve
- 8: Outside air inlet
- 9: Tobacco insertion opening
- 10: Projection
- 11: Control circuit A
- 12: Control circuit B
- 13: Print circuit board
- 21: End sealing portion
- 22: Metal tube
- 23: Magnesium oxide
- 24: Resistance heating wire
- 25: Inner lead wire
- 26: Sleeve case
- 28: Sealing portion
- 29: Bent portion
- 30: End portion
- 41: Outer claw
- 42: Inner claw
- 43: U-shaped stopper
- 44: Stopper positioning knob
- 45: Groove
- 80: Mouthpiece end portion
- 90: Filter cigarette
- 100: Example of the tool of the present application
- 101: Another example of the tool of the present application
- 110: Mouthpiece

## Claims

1. A non-combustion smoking tool comprising one or multiple slender heaters to be inserted into a cigarette or a cigar to directly heat tobacco leaves by the heater so as to vaporize nicotine contained in the tobacco leaves and enable smoker to inhale the nicotine.

2. The non-combustion smoking tool according to claim 1, wherein the heater has an end having a sharp shape and has a diameter of 2.3mm or smaller.

3. The non-combustion smoking tool according to claim 1, wherein the heater is controlled to have a surface temperature of 230°C or lower.
